# EUROPEAN PATENT SPECIFICATION

(11) **EP 4 153 269 B1**
(45) Date of publication and mention of the grant of the patent: **17.06.2026**
(21) Application number: 21721928.6
(22) Date of filing: 29.04.2021
(51) Int. Cl.: A61M 5/142

(54) **SKIN SENSOR TRIGGERING SIGNAL**
HAUTSENSORAUSLÖSESIGNAL
SIGNAL DE DÉCLENCHEMENT D'UN CAPTEUR DE LA PEAU

(30) Priority: 22.05.2020 US 202063028571 P; 16.06.2020 EP 20180345
(43) Date of publication of application: 29.03.2023
(73) Proprietor: SHL Medical AG, 6302 Zug (CH)
(72) Inventor: DEMIROZER, Ramazan, Gurkan, Deerfiled Beach, Florida 33442 (US); STEFANOV, Slobodan, Deerfiled Beach, Florida 33442 (US)
(86) International application number: PCT/EP2021/061266
(87) International publication number: WO 2021/233658

(56) References cited:
- US-A1- 2017 258 987
- US-A1- 2020 061 300
- US-A1- 2020 121 848

## Description

### TECHNICAL FIELD

The present disclosure generally relates to an automated medicament delivery device, and more particularly to such a device having a skin sensor for detecting whether the medicament delivery device has been detached or removed from the skin of the user.

### BACKGROUND

Medicament delivery devices for self-administration have been on the market for a number of years. In order for the devices to be handled by non-professionals, they have to be easy to use and intuitive. Further, since many of the drugs are vital or at least very important to the patient there is a desire from physicians and other professionals to obtain information that the patients medicate according to prescribed schemes. The desired information could include the type of drug, delivery times and dates, dose size. Additional information that could be beneficial to the physician is that the drug has being taken using the correct procedure according to instructions for use; that the drug has the prescribed temperature during drug delivery; that the right injection depth has been used and that the correct injection speed has been used, when the medicament delivery device is an injector.

In order to obtain this information from the medicament delivery device, a number of solutions have been presented, including communication mechanisms which will enable wireless communication with a remote terminal device such as a cellular or a mobile phone. However, the human body has a negative effect on antenna performance. Therefore, it is harder for the medicament delivery device to make a strong wireless connection with a remote terminal device when the medicament delivery device is attached to the user. As such, there is a need to know when the medicament delivery device has been detached from the skin of the user in order to establish a successful wireless communication with a remote device with minimal power dissipation. US 2020/061300 A1 discloses an injection device having a proximity sensor and a wireless transmitter for transmission of treatment log data and other device data to an external server.

### Summary

The present disclosure relates to a device and methods of use for detecting whether a medicament delivery device has been detached or removed from a user such that a signal is triggered and information about the status of the medicament delivery device can be sent.

In particular, the present disclosure provides a medicament delivery device comprising: (a) a housing having a first region and a second region, wherein the housing is configured to receive a medicament container containing a medicament, (b) a delivery needle extending from the second region of the housing, wherein the delivery needle is configured to penetrate a skin of a user to thereby deliver the medicament from the medicament container into the user, (c) a skin sensor positioned at least partially within the housing, (d) a transmitter positioned within the housing, (e) at least one memory storage element including unique identification data associated with the medicament delivery device and/or the user, (f) at least one processor, and (g) data storage including program instructions stored thereon that when executed by the at least one processor, cause the medicament delivery device to: (i) determine, via the skin sensor, whether or not the medicament delivery device is attached to the skin of the user, (ii) in response to a determination that the medicament delivery device is attached to the skin of the user, prevent transmission of the unique identification data from the transmitter, and (iii) in response to a determination that the medicament delivery device is not attached to the skin of the user, transmit, via the transmitter, the unique identification data to one or more external receivers.

These as well as other aspects, advantages, and alternatives, will become apparent to those of ordinary skill in the art by reading the following detailed description, with reference where appropriate to the accompanying drawings.

### BREIF DESCRIPTION OF THE DRAWINGS

Figure 1 illustrates a simplified block diagram of a medicament delivery device, according to an example embodiment.
Figure 2A illustrates a top perspective view of a medicament delivery device, according to an example embodiment.
Figure 2B illustrates a bottom perspective view of the medicament delivery device of Figure 2A, according to an example embodiment.
Figure 3 is a schematic drawing of a wireless communication system, according to an example embodiment.

### DETAILED DESCRIPTION

Example methods and systems are described herein. It should be understood that the words "example," "exemplary," and "illustrative" are used herein to mean "serving as an example, instance, or illustration." Any embodiment or feature described herein as being an "example," being "exemplary," or being "illustrative" is not necessarily to be construed as preferred or advantageous over other embodiments or features. The example embodiments described herein are not meant to be limiting. It will be readily understood that the aspects of the present disclosure, as generally described herein, and illustrated in the figures, can be arranged, substituted, combined, separated, and designed in a wide variety of different configurations, all of which are explicitly contemplated herein.

Furthermore, the particular arrangements shown in the Figures should not be viewed as limiting. It should be understood that other embodiments may include more or less of each element shown in a given Figure. Further, some of the illustrated elements may be combined or omitted. Yet further, an example embodiment may include elements that are not illustrated in the Figures.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

As used herein, apparatus, element and method "configured to" perform a specified function is indeed capable of performing the specified function without any alteration, rather than merely having potential to perform the specified function after further modification. In other words, the apparatus, element, and method "configured to" perform a specified function is specifically selected, created, implemented, utilized, programmed, and/or designed for the purpose of performing the specified function. As used herein, "configured to" refers to existing characteristics of an apparatus, element, and method which enable the apparatus, element, and method to perform the specified function without further modification. For purposes of this disclosure, an apparatus, element, and method described as being "configured to" perform a particular function can additionally or alternatively be described as being "adapted to" and/or as being "operative to" perform that function.

Further, in the following description, the wording medicament delivery device will be used. In this context, medicament delivery devices may include a number of devices capable of delivering certain doses of medicament to a user. The medicament delivery devices may be of either disposable type or re-usable type and may be provided with medicament containers suitably arranged for specific drugs in specific forms.

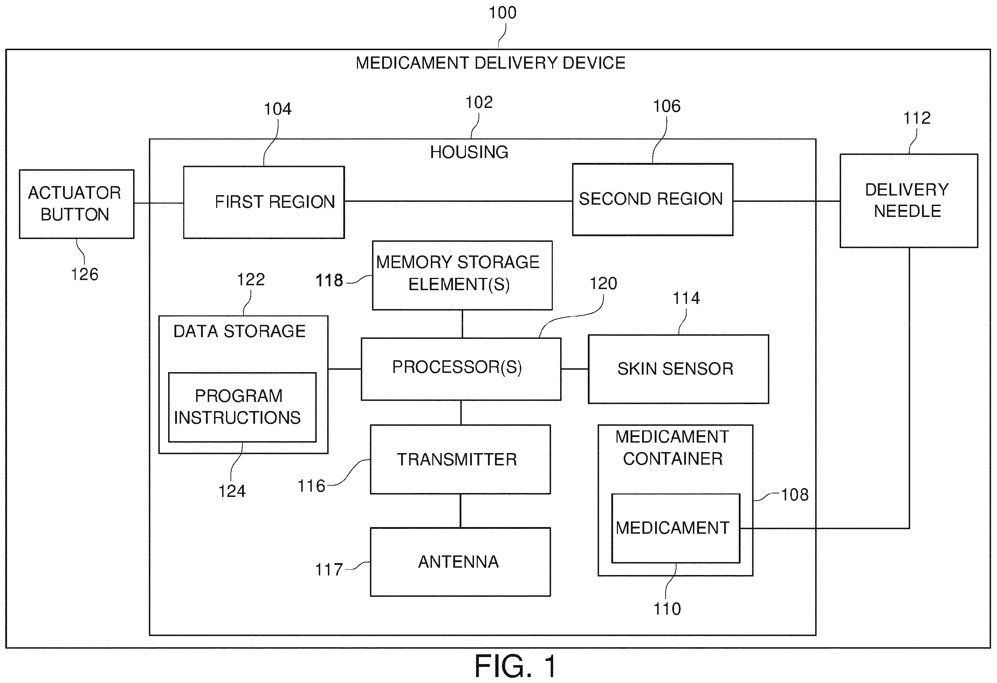

With reference to the figures, Figure 1 illustrates a simplified block diagram of a medicament delivery device 100, according to an example embodiment. As shown in Figure 1, the medicament delivery device 100 includes a housing 102 having a first region 104 and a second region 106 The housing 102 is configured to receive a medicament container 108 containing a medicament 110. In one example the first region 104 is located on one side of the housing 102, and the second region 106 is located on another side of the housing 102. The medicament delivery device 100 also includes a delivery needle 112 extending from the second region 106 of the housing 102. The delivery needle 112 is configured to penetrate a skin of a user to thereby deliver the medicament 110 from the medicament container 108 into the user. The medicament delivery device 100 also includes a skin sensor 114 positioned at least partially within the housing 102, and a transmitter 116 positioned within the housing 102. In some examples, the medicament delivery device 100 also includes an antenna 117 operably connected to the transmitter 116.

As further shown in Figure 1, the medicament delivery device 100 further includes at least one memory storage element 118. The at least one memory storage element 118 includes unique identification data associated with the medicament delivery device and/or the user. Such unique identification data may comprise information including a type of medicament 110 in the medicament delivery device 100, historical delivery times and dates, historical dose sizes, a most recent delivery time and dose size, and additional patient information. The unique identification data may be compared with prescribed drug delivery intervals and/or dose sizes in order to detect any deviations. Any deviations may then be transmitted to the physician of the user for follow up.

The medicament delivery device 100 further includes at least one processor 120, and data storage 122 including program instructions 124 stored thereon that when executed by the at least one processor 120, cause the medicament delivery device 100 to perform functions. In one example, at least one processor may be a field programmable gate array (FPGA).In particular, the functions include (i) determining, via the skin sensor 114, whether or not the medicament delivery device 100 is attached to the skin of the user, (ii) in response to a determination that the medicament delivery device 100 is attached to the skin of the user, prevent transmission of the unique identification data from the transmitter 116, and (iii) in response to a determination that the medicament delivery device 100 is not attached to the skin of the user, transmit, via the transmitter 116, the unique identification data to one or more external receivers. One advantage of the functions (i, ii, iii) is a lower power consumption, as transmittal failure and reattempts for transmitting the unique identification data to one or more external receivers may be reduced. Transmittal failures may can be more likely when the delivery device 100 being attached to the user. The user's body may have a screening effect on the antenna 117. A lower power consumption may eventually have an impact on manufacture costs with reduced power needs, as less, less powerful or smaller parts needed.

In one example, such external receivers may comprise smart devices. In this context, smart devices may include electronic devices that are provided with processors that are capable of running computer programs as well as storage space to store programs as well as data retrieved from different external sources. It is further to be understood that the smart devices are provided with communication systems that are capable of communicating with data networks in order to access different databases. It is to be understood that databases may be accessed via the internet, so called cloud services, and/or databases that are connected directly to and accessed via local area networks. It is further to be understood that the smart devices in this context comprise some sort of human-machine interface for two-way communication. The human-machine interface may comprise displays, keyboards, microphones, loudspeakers, I/O-ports for connection of peripherals. Further the smart devices may be provided with antennas for wireless communication with the networks. Also, the smart devices may be arranged with receiving and transmitting mechanisms capable of communicating with near-field communication (NFC) tags as well as programs capable of establishing and handling the communication with the NFC tags.

The skin sensor 114 may take a variety of forms. In particular, the skin sensor 114 may comprise one or more of a capacitive sensor, a temperature sensor, an ambient light sensor, a pulse oximeter, a heart rate sensor, or an inertial measurement unit (e.g., an accelerometer and gyroscope). The skin sensor 114 is configured to determine whether the medicament delivery device 100 is attached to the skin of the user. The skin sensor 114 may make such a determination constantly, or the skin sensor 114 may make such a determination periodically (e.g., every 1 minutes, every 5 minutes, or every 10 minutes). One advantage of the skin sensor 114 making such determination constantly or periodically may be that as the medicament delivery device 100 may be attached to the user with for example on behalf a wrist band, and a predetermined time laps after the activation button was launched may not determine if the medicament delivery device 100 has been detached from the user. In one example, the skin sensor 114 is configured to contact the skin of the user when the medicament delivery device 100 is attached to the skin of the user. In another example, the skin sensor 114 is configured to be spaced away from the skin of the user when the medicament delivery device 100 is attached to the skin of the user.

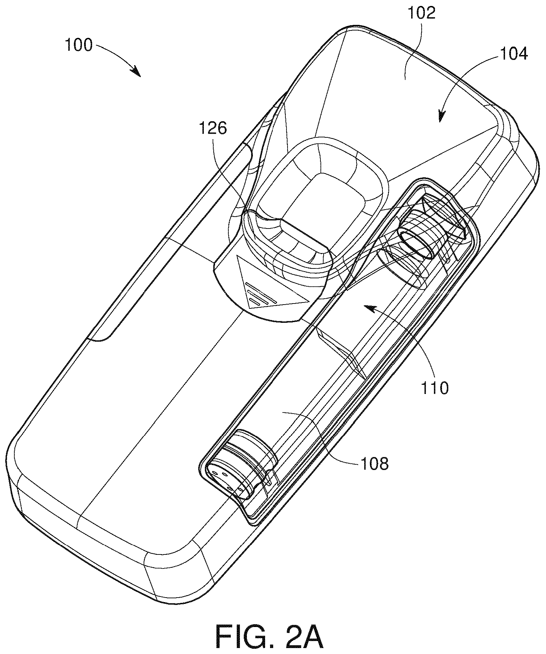

Figure 2A illustrates a top perspective view of the medicament delivery device 100, according to an example embodiment. As shown in Figure 2A, the first region 104 of the housing 102 may include an activation button 126. In use, a user translates the activation button 126 to inject the medicament 110 though the delivery needle 112 and into the user.

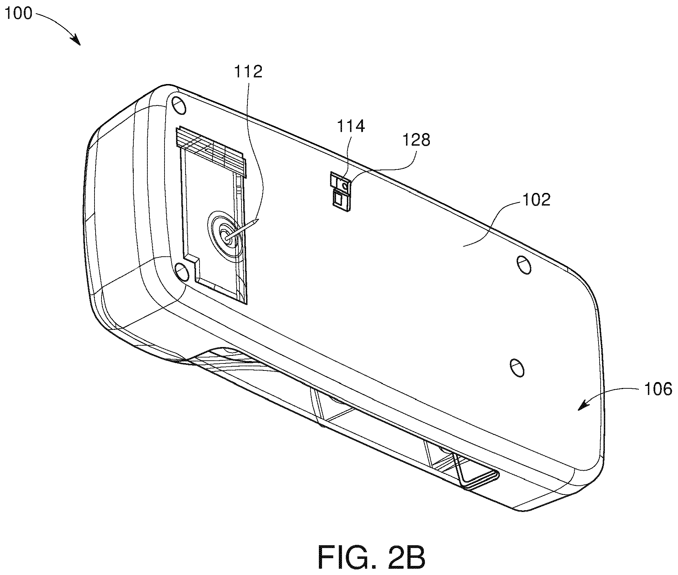

Figure 2B illustrates a bottom perspective view of the medicament delivery device 100 of Figure 2A, according to an example embodiment. As shown in Figure 2B, the second region 106 of the housing 102 may include a cutout 128. In such an example, the skin sensor 114 is positioned in the housing 102 such that the skin sensor 114 is aligned with the cutout 128 in the second region 106 of the housing 102.

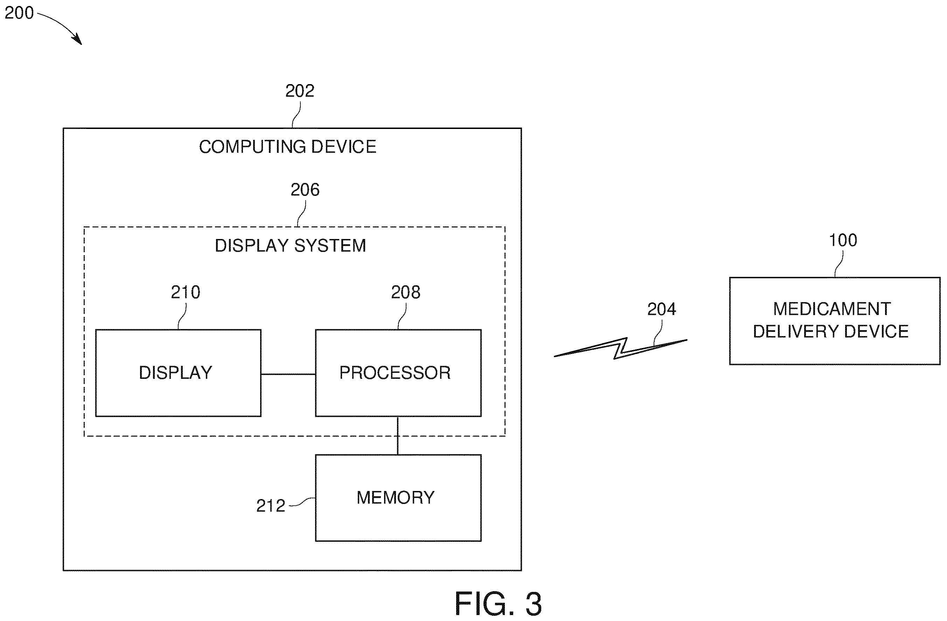

Figure 3 is a schematic drawing of a communication system 200, according to an example embodiment. In the communication system 200, a computing device 202 communicates with the medicament delivery device 100 using a wireless communication link 204. The computing device 202 may be any type of device that can receive data and display information corresponding to or associated with the data. For example, the computing device 202 may be any external receiver, including a smart device (e.g., a mobile phone, a tablet, or a personal computer as non-limiting examples).

Thus, the computing device 202 may include a display system 206 comprising a processor 208 and a display 210. The display 210 may be, for example, an optical see-through display, an optical see-around display, or a video see-through display. In response to a determination that the medicament delivery device 100 is not attached to the skin of the user, the medicament delivery device 100 transmits, via the transmitter 116, unique identification data for receipt by the computing device 202. In particular, the processor 208 may receive the data unique identification data from the medicament delivery device 100, and configure the data for display on the display 210. Depending on the desired configuration, processor 208 can be any type of processor including, but not limited to, a microprocessor, a microcontroller, a digital signal processor, or any combination thereof.

The computing device 202 may further include on-board data storage, such as memory 212 coupled to the processor 208. The memory 212 may store software that can be accessed and executed by the processor 208, for example. The memory 212 can include any type of memory now known or later developed including but not limited to volatile memory (such as RAM), non-volatile memory (such as ROM, flash memory, etc.) or any combination thereof.

According to an example embodiment, the computing device 202 may include program instructions that are stored in the memory 212 (and/or possibly in another data-storage medium) and executable by the processor 208 to facilitate the various functions described herein. Although various components of the communication system 200 are shown as distributed components, it should be understood that any of such components may be physically integrated and/or distributed according to the desired configuration of the computing system.

The medicament delivery device 100 and the computing device 202 may contain hardware to enable the wireless communication link 204, such as processors, transmitters, receivers, antennas, etc., as discussed above.

In one example, the wireless communication link 204 may comprise near-field communication (NFC) technology, Bluetooth^{®} radio technology, communication protocols described in IEEE 802.11 (including any IEEE 802.11 revisions), Cellular technology (such as GSM, CDMA, UMTS, EV-DO, WiMAX, or LTE), or Zigbee^{®} technology, among other possibilities.

In use, the skin sensor 114 detects (either constantly or periodically) whether or not the medicament delivery device 100 is attached to the skin of the user. In one example, the skin sensor 114 begins detecting whether or not the medicament delivery device 100 is attached to the skin of the user after the medicament delivery device 100 determines that a dose of medicament 110 from the medicament container 108 has been delivered to the user. If a determination is made that the medicament delivery device 100 is still attached to the skin of the user, transmission of unique identification data from the transmitter 116 is prevented. However, if a determination is made that the medicament delivery device 100 is not attached to the skin of the user, the unique identification data is transmitted from the transmitter 116 to one or more external receivers. Such a process helps to establish a successful wireless communication between the medicament delivery device 100 and a remote device with minimal power dissipation.

It will be appreciated that other arrangements are possible as well, including some arrangements that involve more or fewer steps than those described above, or steps in a different order than those described above.

While various aspects and embodiments have been disclosed herein, other aspects and embodiments will be apparent to those skilled in the art. All embodiments within and between different aspects of the devices and methods can be combined unless the context clearly dictates otherwise. The various aspects and embodiments disclosed herein are for purposes of illustration and are not intended to be limiting, with the true scope of the invention being indicated by the claims.

## Claims

1. A medicament delivery device (100) comprising:
a housing (102) having a first region (104) and a second region (106), wherein the housing (102) is configured to receive a medicament container (108) containing a medicament (110);
a delivery needle (112) extending from the second region (106) of the housing (102), wherein the delivery needle (112) is configured to penetrate a skin of a user to thereby deliver the medicament (110) from the medicament container (108) into the user;
a skin sensor (114) positioned at least partially within the housing (102);
a transmitter (116) positioned within the housing (102);
at least one memory storage element (118) including unique identification data associated with the medicament delivery device and/or the user;
at least one processor (120); **characterized in that** the medicament delivery device (100)
further comprises:
data storage (122) including program instructions (124) stored thereon that when executed by the at least one processor (120), cause the medicament delivery device (100) to:
determine, via the skin sensor (114), whether or not the medicament delivery device (100) is attached to the skin of the user;
in response to a determination that the medicament delivery device (100) is attached to the skin of the user, prevent transmission of the unique identification data from the transmitter (116); and
in response to a determination that the medicament delivery device (100) is not attached to the skin of the user, transmit (204), via the transmitter (116), the unique identification data to one or more external receivers (200).

2. The medicament delivery (100) device of claim 1, wherein the skin sensor (114) comprises one or more of a capacitive sensor, a temperature sensor, an ambient light sensor, a pulse oximeter, a heart rate sensor, or an inertial measurement unit.

3. The medicament delivery device (100) of any one of claims 1-2, wherein the second region (106) of the housing (102) includes a cutout (128).

4. The medicament delivery device (100) of claim 3, wherein the skin sensor (114) is positioned in the housing (102) such that the skin sensor (114) is aligned with the cutout (128) in the second region (106) of the housing.

5. The medicament delivery device (100) of any one of claims 1-4, wherein the skin sensor (114) is configured to contact the skin of the user when the medicament delivery device (100) is attached to the skin of the user.

6. The medicament delivery device (100) of any one of claims 1-4, wherein the skin sensor (114) is configured to be spaced away from the skin of the user when the medicament delivery device (100) is attached to the skin of the user.

7. The medicament delivery device (100) of any one of claims 1-6, further comprising an activation button (126) positioned on the first region (104) of the housing (102).

8. The medicament delivery device (100) of any one of claims 1-7, further comprising an antenna (117) operably connected to the transmitter (116).

9. The medicament delivery device (100) of any one of claims 1-8, wherein the first region (104) is located on one side of the housing (102) and the second region (106) is located on another side of the housing (102).

## Patentansprüche

1. Medikamentenabgabevorrichtung (100), umfassend:
ein Gehäuse (102), das einen ersten Bereich (104) und einen zweiten Bereich (106) aufweist, wobei das Gehäuse (102) konfiguriert ist, um einen Medikamentenbehälter (108) aufzunehmen, der ein Medikament (110) enthält;
eine Abgabenadel (112), die sich von dem zweiten Bereich (106) des Gehäuses (102) erstreckt, wobei die Abgabenadel (112) konfiguriert ist, um eine Haut eines Benutzers zu durchdringen, um dadurch das Medikament (110) aus dem Medikamentenbehälter (108) in den Benutzer abzugeben;
ein Hautsensor (114), der mindestens teilweise innerhalb des Gehäuses (102) positioniert ist;
einen Transmitter (116), der innerhalb des Gehäuses (102) positioniert ist;
mindestens ein Speicherelement (118), das eindeutige Identifikationsdaten einschließt, die der Medikamentenabgabevorrichtung und/oder dem Benutzer zugeordnet sind;
mindestens einen Prozessor (120); **dadurch gekennzeichnet, dass** die Medikamentenabgabevorrichtung (100) ferner umfasst:
Datenspeicher (122), der Programmanweisungen (124) einschließt, die darauf gespeichert sind, die, wenn sie durch den mindestens einen Prozessor (120) ausgeführt werden, die Medikamentenabgabevorrichtung (100) veranlassen zum:
Bestimmen, über den Hautsensor (114), ob die Medikamentenabgabevorrichtung (100) an der Haut des Benutzers angebracht ist oder nicht;
als Reaktion auf eine Bestimmung, dass die Medikamentenabgabevorrichtung (100) an der Haut des Benutzers angebracht ist, Übertragung der eindeutigen Identifikationsdaten von dem Transmitter (116) verhindern; und
als Reaktion auf eine Bestimmung, dass die Medikamentenabgabevorrichtung (100) nicht an der Haut des Benutzers angebracht ist, Übertragen (204) über den Transmitter (116) der eindeutigen Identifikationsdaten an einen oder mehrere externe Empfänger (200).

2. Medikamentenabgabe(100)-Vorrichtung nach Anspruch 1, wobei der Hautsensor (114) einen oder mehrere von einem kapazitiven Sensor, einem Temperatursensor, einem Umgebungslichtsensor, einem Pulsoximeter, einem Herzfrequenzsensor oder einer Trägheitsmesseinheit umfasst.

3. Medikamentenabgabevorrichtung (100) nach einem der Ansprüche 1 bis 2, wobei der zweite Bereich (106) des Gehäuses (102) einen Ausschnitt (128) einschließt.

4. Medikamentenabgabevorrichtung (100) nach Anspruch 3, wobei der Hautsensor (114) in dem Gehäuse (102) positioniert ist, derart, dass der Hautsensor (114) mit dem Ausschnitt (128) in dem zweiten Bereich (106) des Gehäuses ausgerichtet ist.

5. Medikamentenabgabevorrichtung (100) nach einem der Ansprüche 1 bis 4, wobei der Hautsensor (114) konfiguriert ist, um die Haut des Benutzers zu kontaktieren, wenn die Medikamentenabgabevorrichtung (100) an der Haut des Benutzers angebracht ist.

6. Medikamentenabgabevorrichtung (100) nach einem der Ansprüche 1 bis 4, wobei der Hautsensor (114) konfiguriert ist, um von der Haut des Benutzers weg beabstandet zu sein, wenn die Medikamentenabgabevorrichtung (100) an der Haut des Benutzers angebracht ist.

7. Medikamentenabgabevorrichtung (100) nach einem der Ansprüche 1 bis 6, ferner umfassend einen Aktivierungsknopf (126), der auf dem ersten Bereich (104) des Gehäuses (102) positioniert ist.

8. Medikamentenabgabevorrichtung (100) nach einem der Ansprüche 1 bis 7, ferner umfassend eine Antenne (117), die mit dem Transmitter (116) wirkverbunden ist.

9. Medikamentenabgabevorrichtung (100) nach einem der Ansprüche 1 bis 8, wobei sich der erste Bereich (104) auf einer Seite des Gehäuses (102) befindet und sich der zweite Bereich (106) auf einer anderen Seite des Gehäuses (102) befindet.

## Revendications

1. Dispositif d'administration de médicament (100) comprenant :
un logement (102) ayant une première région (104) et une seconde région (106), dans lequel le logement (102) est conçu pour recevoir un récipient de médicament (108) contenant un médicament (110) ;
une aiguille d'administration (112) s'étendant à partir de la seconde région (106) du logement (102), dans lequel l'aiguille d'administration (112) est conçue pour pénétrer dans la peau d'un utilisateur afin d'administrer de ce fait le médicament (110) à partir du récipient de médicament (108) à l'utilisateur ;
un capteur de peau (114) positionné au moins partiellement à l'intérieur du logement (102) ;
un émetteur (116) positionné à l'intérieur du logement (102) ;
au moins un élément de stockage de mémoire (118) comportant des données d'identification uniques associées au dispositif d'administration de médicament et/ou à l'utilisateur ;
au moins un processeur (120) ; **caractérisé en ce que** le dispositif d'administration de médicament (100) comprend en outre :
un stockage de données (122) comportant des instructions de programme (124) stockées sur celui-ci qui, lorsqu'elles sont exécutées par l'au moins un processeur (120), amènent le dispositif d'administration de médicament (100) à :
déterminer, par l'intermédiaire du capteur de peau (114), si le dispositif d'administration de médicament (100) est ou non fixé à la peau de l'utilisateur ;
en réponse à une détermination que le dispositif d'administration de médicament (100) est fixé à la peau de l'utilisateur, empêcher la transmission des données d'identification uniques à partir de l'émetteur (116) ; et
en réponse à la détermination que le dispositif d'administration de médicament (100) n'est pas fixé à la peau de l'utilisateur, transmettre (204), par l'intermédiaire de l'émetteur (116), les données d'identification uniques à un ou plusieurs récepteurs externes (200).

2. Dispositif d'administration de médicament (100) selon la revendication 1, dans lequel le capteur de peau (114) comprend un ou plusieurs parmi un capteur capacitif, un capteur de température, un capteur de lumière ambiante, un oxymètre de pouls, un capteur de fréquence cardiaque ou une unité de mesure inertielle.

3. Dispositif d'administration de médicament (100) selon l'une quelconque des revendications 1 à 2, dans lequel la seconde région (106) du logement (102) comporte une découpe (128).

4. Dispositif d'administration de médicament (100) selon la revendication 3, dans lequel le capteur de peau (114) est positionné dans le logement (102) de telle sorte que le capteur de peau (114) est aligné avec la découpe (128) dans la seconde région (106) du logement.

5. Dispositif d'administration de médicament (100) selon l'une quelconque des revendications 1 à 4, dans lequel le capteur de peau (114) est configuré pour entrer en contact avec la peau de l'utilisateur lorsque le dispositif d'administration de médicament (100) est fixé à la peau de l'utilisateur.

6. Dispositif d'administration de médicament (100) selon l'une quelconque des revendications 1 à 4, dans lequel le capteur de peau (114) est configuré pour être éloigné de la peau de l'utilisateur lorsque le dispositif d'administration de médicament (100) est fixé à la peau de l'utilisateur.

7. Dispositif d'administration de médicament (100) selon l'une quelconque des revendications 1 à 6, comprenant en outre un bouton d'activation (126) positionné sur la première région (104) du logement (102).

8. Dispositif d'administration de médicament (100) selon l'une quelconque des revendications 1 à 7, comprenant en outre une antenne (117) connectée de manière opérationnelle à l'émetteur (116).

9. Dispositif d'administration de médicament (100) selon l'une quelconque des revendications 1 à 8, dans lequel la première région (104) est située sur un côté du logement (102) et la seconde région (106) est située sur un autre côté du logement (102).
